# FASCICULE DE BREVET EUROPEEN

(11) **EP 0 663 906 B1**
(45) Date de publication et mention de la délivrance du brevet: **11.12.1996**
(21) Numéro de dépôt: 93921982.0
(22) Date de dépôt: 04.10.1993
(51) Int. Cl.: C07D 305/14, C07D 263/06, C07D 413/12

(54) **PROCEDE DE PREPARATION DE DERIVES DU TAXANE**
VERFAHREN FÜR DIE HERSTELLUNG VON TOXANE DERIVATEN
METHOD OF PREPARING TAXANE DERIVATIVES

(30) Priorité: 05.10.1992 FR 9211741
(43) Date de publication de la demande: 26.07.1995
(73) Titulaire: RHONE-POULENC RORER S.A., 92165 Antony Cédex (FR)
(72) Inventeur: MAS, Jean-Manuel, F-69100 Villeurbanne (FR); MASSONNEAU, Viviane Charrière Blanche, F-69130 Ecully (FR)
(74) Mandataire: Pilard, Jacques
(86) Numéro de dépôt international: FR9300967
(87) Numéro de publication internationale: WO9407877

(56) Documents cités:
- EP-A- 0 400 971
- EP-A- 0 428 376
- WO-A-92/09589
- WO-A-93/23389
- J. MED. CHEM. vol. 34, no. 3, Mars 1991, WASHINGTON US pages 992 - 998 F. GUERITTE-VOEGELEIN ET AL.
- STUD. ORG. CHEM. (NEW TRENDS IN NAT. PROD. CHEM. 1986) vol. 26, 1986, AMSTERDAM
- pages 219 - 235 D.G.I. KINGSTON ET AL.

## Description

La présente invention concerne un nouveau procédé de préparation de dérivés du taxane de formule générale : 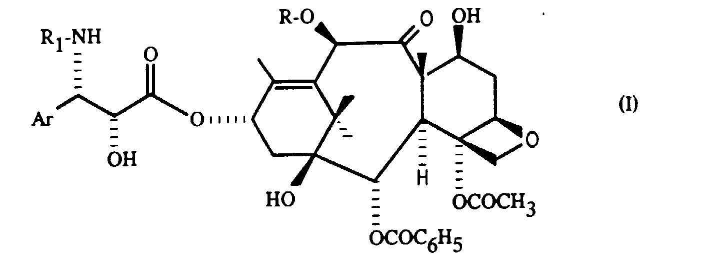 qui présentent des propriétés antileucémiques et antitumorales remarquables.

Dans la formule générale (I) :
R représente un atome d'hydrogène ou un radical acétyle, R₁ représente un radical benzoyle ou un radical R₂-O-CO- dans lequel R₂ représente un radical alcoyle, alcényle, alcynyle, cycloalcoyle, cycloalcényle, bicycloalcoyle, phényle ou hétéro-cyclyle azoté, et Ar représente un radical aryle.

Plus particulièrement, R représente un atome d'hydrogène ou un radical acétyle et R₁ représente un radical benzoyle ou un radical R₂-O-CO- dans lequel R₂ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en 4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les radicaux alcoyles contenant 1 à 4 atomes de carbone ou alcoyloxy contenant 1 à 4 atomes de carbone,
- ou un radical hétérocyclique azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alkylamino, dialkylamino, carboxy, alcoxycarbonyle, carbamoyle, dialcoylcarbamoyle, cyano et trifluorométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et les radicaux aryles sont les radicaux phényles ou α- ou β-naphtyles.

D'un intérêt tout particulier sont les produits de formule générale (I) dans laquelle R représente un atome d'hydrogène ou un radical acétyle, R₁ représente un radical benzoyle ou t.butoxycarbonylamino et Ar représente un radical phényle.

Les produits de formule générale (I) dans laquelle R₁ représente un radical benzoyle correspondent au taxol et au désacétyl-10 taxol et les produits de formule générale (I) dans laquelle R₁ représente un radical t.butoxycarbonyle correspondent à ceux qui font l'objet du brevet européen EP 0 253 738.

Selon le procédé qui est décrit dans la demande internationale PCT WO 92/09589, les dérivés de formule générale (I) peuvent être obtenus par :
- condensation d'un dérivé de l'oxazolidine de formule générale : 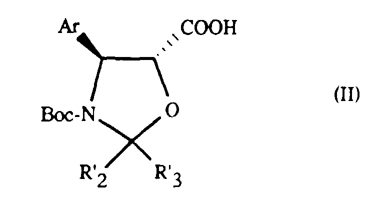 dans laquelle Ar est défini comme précédemment, Boc représente le radical t.butoxycarbonyle et R'₂ et R'₃, identiques ou différents, représentent un radical alcoyle contenant 1 à 4 atomes de carbone éventuellement substitué par un ou plusieurs radicaux aryles, ou un radical aryle, ou bien R'₂ et R'₃ forment ensemble avec l'atome de carbone auquel ils sont liés un cycle ayant de 4 à 7 chaînons, sur la baccatine III ou la désacétyl-10 baccatine III protégée de formule générale : 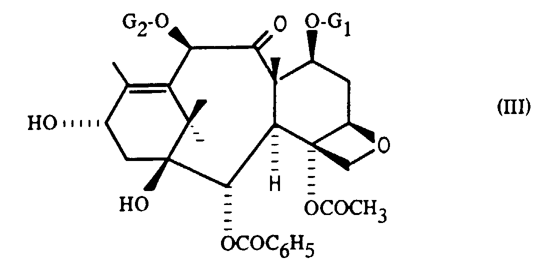 dans laquelle G₁ représente un groupement protecteur de la fonction hydroxy et G₂ représente un radical acétyle ou un groupement protecteur de la fonction hydroxy, pour obtenir un produit de formule générale : 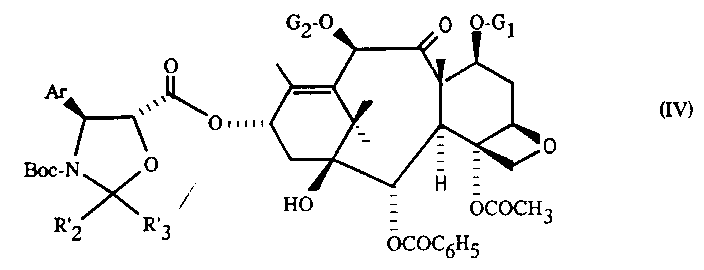 dans laquelle Ar, R'₂, R'₃, G₁, G₂ et Boc sont définis comme précédemment,
- traitement en milieu acide du produit de formule générale (IV) dans des conditions qui sont sans effet sur G₁ et G₂ pour obtenir le produit de formule générale : 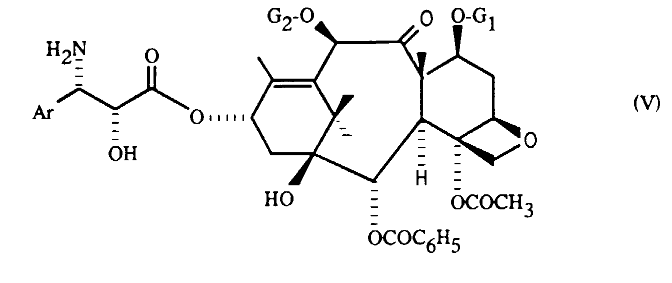 dans laquelle Ar, G₁ et G₂ sont définis comme précédemment,
- traitement du produit de formule générale (V) par un réactif convenable pour introduire un radical benzoyle ou R₂-O-CO-, pour obtenir un produit de formule générale : 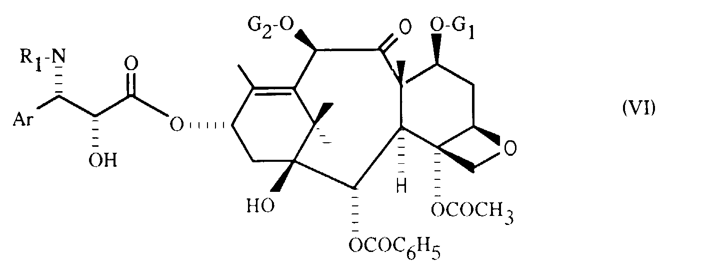 dans laquelle Ar, R₁, G₁ et G₂ sont définis comme précédemment, et
- remplacement des groupements protecteurs G₁ et G₂ du produit de formule générale (VI) par des atomes d'hydrogène pour obtenir le produit de formule générale (I).

Il a maintenant été trouvé, et c'est ce qui fait l'objet de la présente invention, que les produits de formule générale (I) peuvent être obtenus :
1) en estérifiant la baccatine III ou la désacétyl-10 baccatine III de formule générale (III) dans laquelle G₁ et éventuellement G₂ représentent un groupement protecteur de la fonction hydroxy, de préférence, un radical trialkylsilyle, dialkylarylsilyle, alkyldiarylsilyle ou triarylsilyle et plus particulièrement encore un radical R₅-O-CO- dans lequel R₅ est défini ci-après au moyen d'un acide de formule générale : 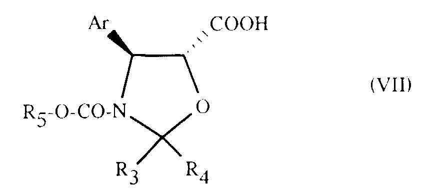 dans laquelle Ar est défini comme précédemment, R₃ et R₄, identiques ou différents, représentent un atome d'hydrogène ou un radical alcoyle contenant 1 à 4 atomes de carbone, ou un radical alcényle contenant 2 à 4 atomes de carbone, ou un radical aralcoyle dont la partie alcoyle contient 1 à 4 atomes de carbone et la partie aryle représente un radical phényle éventuellement substitué par un ou plusieurs radicaux alcoxy contenant 1 à 4 atomes de carbone, ou un radical aryle représentant un radical phényle éventuellement substitué par un ou plusieurs radicaux alcoxy contenant 1 à 4 atomes de carbone, ou bien R₃ et R₄ forment ensemble avec l'atome de carbone auquel ils sont liés un cycle ayant de 4 à 7 chaînons, étant entendu que R₃ et R₄ ne représentent pas simultanément un atome d'hydrogène et que lorque l'un des symboles R₃ et R₄ représente un atome d'hydrogène, l'autre ne peut pas représenter un radical phényle substitué par un ou plusieurs radicaux alcoxy contenant 1 à 4 atomes de carbone ou par un radical trihalométhyle et R₅ représente un radical alcoyle contenant 1 à 4 atomes de carbone substitué par un ou plusieurs atomes de chlore, pour obtenir un produit de formule générale: 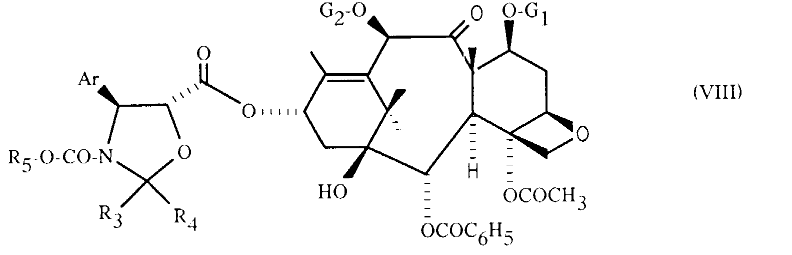 dans laquelle Ar, R₃, R₄, R₅, G₁ et G₂ sont définis comme précédemment.
2) en remplaçant les groupements protecteurs des fonctions hydroxy et amino du produit obtenu de formule générale (VIII) par des atomes d'hydrogène pour obtenir le produit de formule générale : 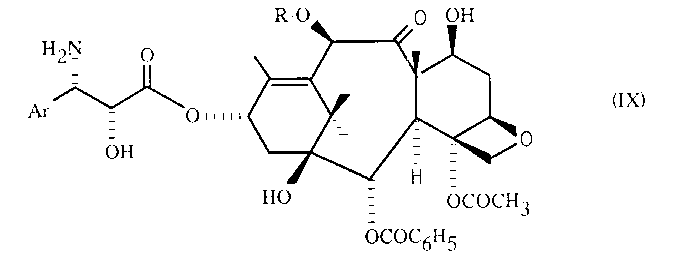 dans laquelle Ar et R sont définis comme précédemment,
3) en traitant le produit obtenu de formule générale (IX) par un réactif qui permet d'introduire un substituant R₁ sur la fonction amino pour obtenir un produit de formule générale (I).

Les produits de formule générale (IX) peuvent aussi être obtenus par un procédé mettant en oeuvre une réaction d'oxyamination vicinale de Sharpless telle que décrite dans J.Med.Chem., 24(3) 992 (1991).

Selon la présente invention, l'estérification de la baccatine III ou de la désacétyl-10 baccatine III protégée de formule générale (III) au moyen d'un acide de formule générale (VII) dans laquelle R₅ représente de préférence un radical trichloro-2,2,2 éthyle ou trichlorométhyl-2 isopropyle, peut être effectuée en présence d'un agent de condensation tel qu'un diimide comme le dicyclohexylcarbodiimide ou un carbonate réactif comme le dipyridyl-2 carbonate et d'un agent d'activation tel qu'une aminopyridine comme la diméthylamino-4 pyridine ou la pyrrolidino-4 pyridine en opérant dans un solvant organique choisi parmi les éthers tels que le tétrahydrofuranne, l'éther diisopropylique, le méthyl t.butyléther ou le dioxanne, les cétones telles que la méthylisobutylcétone, les esters tels que l'acétate d'éthyle, l'acétate d'isopropyle, l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane et les hydrocarbures aromatiques tels que le benzène, le toluène, les xylènes, l'éthylmnùne, l'isopropylbenzène ou le chlorobenzène à une température comprise entre -10 et 90°C. Il est particulièrement avantageux d'effectuer l'estérification en opérant dans un hydrocarbure aromatique à une température voisine de 20°C.

L'estérification peut aussi être réalisée en utilisant l'acide de formule générale (VII) sous forme d'anhydride de formule générale : 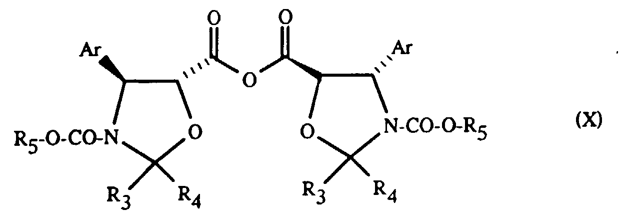 dans laquelle Ar, R₃, R₄ et R₅ sont définis comme précédemment, R₅ représentant de préférence un radical trichloro-2,2,2 éthyle ou trichlorométhyl-2 isopropyle, en présence d'un agent d'activation tel qu'une aminopyridine comme la diméthylamino-4 pyridine ou la pyrrolidino-4 pyridine en opérant dans un solvant organique choisi parmi les éthers tels que le tétrahydrofuranne, le diisopropyléther, le méthyl t.butyléther ou le dioxanne, les cétones telles que la méthylisobutylcétone, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures halogénés tels que le dichlorométhane ou le dichloro-1,2 éthane et les hydrocarbures aromatiques tels que le benzène, le toluène, les xylènes, l'éthylbenzène, l'isopropylbenzène ou le chlorobenzène à une température comprise entre 0 et 90°C.

L'estérification peut aussi être réalisée en utilisant l'acide de formule générale (VII) sous forme d'halogénure ou d'anhydride mixte de formule générale : 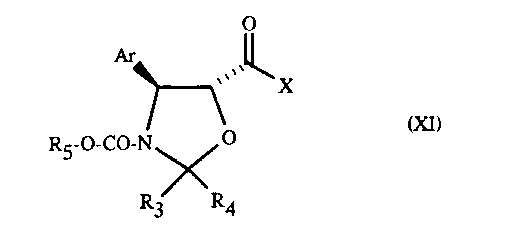 dans laquelle Ar, R₃, R₄ et R₅ sont définis comme précédemment, R₅ représentant de préférence un radical trichloro-2,2,2 éthyle ou trichlorométhyl-2 isopropyle, et X représente un atome d'halogène ou un radical acyloxy ou aroyloxy, éventuellement préparé in situ, en présence d'une base qui est de préférence une base organique azotée telle qu'une amine aliphatique tertiaire comme la triéthylamine, la pyridine ou une aminopyridine comme la diméthylamino-4 pyridine ou la pyrrolidino-4 pyridine en opérant dans un solvant organique inerte choisi parmi les éthers tels que le tétrahydrofuranne, l'éther diisopropylique, le méthyl t.butyléther ou le dioxanne, les cétones, les esters tels que l'acétate d'éthyle, l'acétate d'isopropyle ou l'acétate de n.butyle, les nitriles tels que l'acétonitrile, les hydrocarbures aliphatiques tels que le pentane, l'hexane ou l'heptane, les hydrocarbures aliphatiques halogénés tels que le dichlorométhane ou le dichloro-1,2 éthane et les hydrocarbures aromatiques tels que le benzène, le toluène, les xylènes, l'éthylbenzène, l'isopropylbenzène ou le chlorobenzène à une température comprise entre 10 et 80°C, de préférence voisine de 20°C.

De préférence, on utilise un dérivé activé de formule générale (XI) dans laquelle X représente un atome d'halogène ou un radical acyloxy contenant 1 à 5 atomes de carbone, aroyloxy dans lequel la partie aryle est un radical phényle éventuellement substitué par 1 à 5 atomes ou radicaux, identiques ou différents, choisis parmi les atomes d'halogène (chlore, brome) et les radicaux nitro, méthyle ou méthoxy.

Le remplacement par des atomes d'hydrogène des groupements protecteurs des fonctions hydroxy et amino du produit de formule générale (VIII), dans laquelle R₅ représente, de préférence, un radical trichloro-2,2,2 éthyle ou (trichlorométhyl-2 propyle)-2, G₁ et éventuellement G₂ représentent un groupement protecteur de la fonction hydroxy, de préférence un radical trichloro-2,2,2 éthoxycarbonyle ou (trichlorométhyl-2 propoxy)-2 carbonyle et est effectué généralement par traitement par le zinc, éventuellement associé à du cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone ou dans un ester aliphatique tel que l'acétate d'éthyle, l'acétate d'isopropyle ou l'acétate de n.butyle en présence de zinc éventuellement associé à du cuivre.

Le remplacement par des atomes d'hydrogène des groupements protecteurs des fonctions hydroxy et amino du produit de formule générale (VIII), dans laquelle R₅ représente , de préférence, un radical trichloro-2,2,2 éthyle ou (trichlorométhyl-2 propyle)-2, G₁ et éventuellement G₂ représentent un groupement protecteur de la fonction hydroxy, de préférence, un radical trialkylsilyle, dialkylarylsilyle, alkyldiarylsilyle ou triarylsilyle est effectué généralement par traitement en milieu acide tel que par exemple l'acide chlorhydrique en solution dans un alcool aliphatique contenant 1 à 3 atomes de carbone (méthanol, éthanol, propanol, isopropanol) ou l'acide fluorhydrique aqueux à une température comprise entre 0 et 40°C, pour remplacer les groupements protecteurs G₁ et éventuellement G₂, et par traitement par le zinc, éventuellement associé à du cuivre, en présence d'acide acétique à une température comprise entre 30 et 60°C ou au moyen d'un acide minéral ou organique tel que l'acide chlorhydrique ou l'acide acétique en solution dans un alcool aliphatique contenant 1 à 3 atomes de carbone ou un ester aliphatique tel que l'acétate d'éthyle, l'acétate d'isopropyle ou l'acétate de n.butyle en présence de zinc éventuellement associé à du cuivre pour remplacer R₅.

Le remplacement des groupements protecteurs du produit de formule générale (VIII) par des atomes d'hydrogène peut être également effectué par réduction électrolytique.

L'introduction d'un substituant R₁ sur la fonction amino du produit de formule générale (IX) est effectuée par action du chlorure de benzoyle ou d'un dérivé réactif de formule générale :

R₂-O-CO-Y (XII)

dans laquelle R₂ est défini comme précédemment et Y représente un atome d'halogène (fluor, chlore) ou un reste -O-R₂ ou -O-CO-OR₂ en opérant dans un solvant organique tel qu'un alcool comme le méthanol, un ester aliphatique comme l'acétate d'éthyle ou un hydrocarbure aliphatique halogéné comme le dichlorométhane en présence d'une base minérale ou organique telle que le bicarbonate de sodium. Généralement la réaction est effectuée à une température comprise entre 0 et 50°C, de préférence voisine de 20°C.

L'acide de formule générale (VII) peut être obtenu par saponification en milieu basique de l'ester de formule générale : 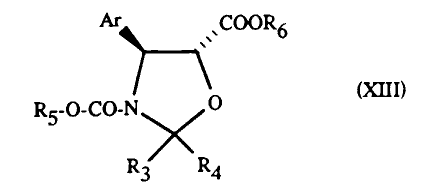 dans laquelle Ar, R₃, R4 et R₅ sont définis comme précédemment et R₆ représente un radical alcoyle contenant 1 à 4 atomes de carbone éventuellement substitué par un radical phényle.

Généralement, la saponification est effectuée au moyen d'une base minérale telle qu'un hydroxyde de métal alcalin (lithium, potassium, sodium), un carbonate ou bicarbonate de métal alcalin (bicarbonate de sodium, carbonate ou bicarbonate de potassium) en milieu hydro-alcoolique tel qu'un mélange méthanol-eau à une température comprise entre 10 et 40°C, de préférence voisine de 20°C.

L'ester de formule générale (XIII) peut être obtenu par action d'un produit de formule générale : 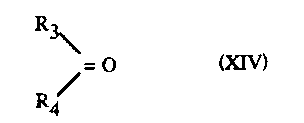 dans laquelle R₃ et R₄ sont définis comme précédemment éventuellement sous forme d'un dialkylacétal ou d'un alkyléther d'énol, sur un dérivé de la phénylisosérine de formule générale : 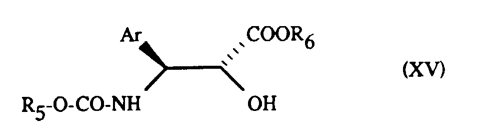 dans laquelle Ar, R₅ et R₆ sont définis comme précédemment, sous forme racémique ou, de préférence, sous forme 2R,3S en opérant dans un solvant organique inerte en présence d'un acide fort minéral, tel que l'acide sulfurique, ou organique tel que l'acide p.toluènesulfonique éventuellement sous forme de sel de pyridinium à une température comprise entre 0°C et la température d'ébullition du mélange réactionnel. Les solvants qui conviennent particulièrement bien sont les hydrocarbures aromatiques.

Le produit de formule générale (XV) peut être préparé dans les conditions décrites dans la demande internationale PCT WO 92/09589.

L'anhydride de formule générale (X) peut être obtenu en faisant réagir un agent de déshydratation tel que le dicyclohexylcarbodiimide sur l'acide de formule générale (VII) en opérant dans un solvant organique choisi parmi les hydrocarbures aliphatiques halogénés et les hydrocarbures aromatiques à une température comprise entre 0 et 30°C.

L'acide activé de formule générale (XI) peut être obtenu par action d'un halogénure de sulfuryle, de préférence le chlorure, ou d'un produit de formule générale :

R₇-CO-Z (XVI)

dans laquelle R₇ représente un radical alcoyle contenant 1 à 4 atomes de carbone ou un radical phényle éventuellement substitué par 1 à 5 atomes ou radicaux, identiques ou différents, choisis parmi les atomes d'halogène et les radicaux nitro, méthyle ou méthoxy et Z représente un atome d'halogène, de préférence un atome de chlore, sur un acide de formule générale (VII) en opérant dans un solvant organique convenable tel que le tétrahydrofuranne en présence d'une base organique telle qu'une amine tertiaire comme la triéthylamine à une température comprise entre 0 et 30°C. Les exemples suivants illustrent la présente invention.

### EXEMPLE 1

A une solution de 11,7 g (60 mmoles) de phénylisosérinate (2R,3S) de méthyle et de 5,22 g de pyridine dans 180 cm3 de chlorure de méthylène on ajoute, en 50 minutes, à 0°C, 9,48 cm3 de chlorure de trichloro-2,2,2 éthoxycarbonyle. On laisse revenir la température du mélange réactionnel remonter au voisinage de 20°C tout en agitant pendant 3 heures. La solution est lavée par 100 cm3 d'une solution aqueuse d'acide chlorhydrique 0,1N puis par 2 fois 50 cm3 d'eau. Après séchage et concentration sous pression réduite de la phase organique, le résidu est repris par 300 cm3 de cyclohexane. Le solvant est alors concentré partiellement sous pression réduite (60 kPa) à 40°C jusqu'à apparition des premiers cristaux. Le précipité ainsi obtenu est isolé par filtration puis lavé au cyclohexane et séché. On obtient ainsi, avec un rendement de 86%, 19,1 g d'hydroxy-2 phényl-3 (trichloro-2,2,2 éthoxycarbonylamino)-3 propionate (2R,3S) de méthyle dont les caractéristiques sont les suivantes :
- spectre de RMN du proton (360 MHz ; DMSO-d₆ ; déplacements chimiques en ppm ; constantes de couplage J en Hz) : 8,12 (d, J = 9,2, 1H); 7,20 (M, 5H) ; 5,63 (M, 1H); 4,89 (dd, J = 5,1 et 9,2, 1H); 4,77 et 4,67 (syst AB, J = -12,3, 1H); 4,29 (m, 1H); 3,46 (s, 3H).

A une solution de 11,1 g (30 mmoles) d'hydroxy-2 phényl-3 (trichloro-2,2,2 éthoxycarbonylamino)-3 propionate (2R,3S) de méthyle et de 3,24 g de méthoxy-2 propène dans 100 cm3 de toluène on ajoute 151 mg de paratoluène sulfonate de pyridinium. Le mélange réactionnel est chauffé jusqu'à ébullition. On ajoute en 2 heures 50 cm3 d'une solution toluénique contenant 19,5 g de méthoxy-2 propène. On effectue une distillation jusqu'à l'obtention de 80 cm3 de distillat. Le mélange réactionnel est refroidi à une température voisine de 20°C, traité au bicarbonate de sodium puis lavé à l'eau. La solution organique est séchée puis concentrée sous pression réduite. On obtient ainsi 14,5 g de produit brut huileux qui est chromatographié sur une colonne de silice en éluant avec un mélange cyclohexane/acétate d'éthyle (90-10 en volumes). On isole 5,68 g de méthoxycarbonyl-5 diméthyl-2,2 phényl-4 (trichloro-2,2,2 éthoxycarbonyl)-3 oxazolidine-1,3 (4S,5R) pur (rendement = 46 %) et 4,95 g de méthoxycarbonyl-5 méthyl-2 isobutényl-2 phényl-4 (trichloro-2,2,2 éthoxycarbonyl)-3 oxazolidine-1,3 (4S,5R) pur (rendement = 36,7 %).

### EXEMPLE 2

On ajoute une solution méthanolique de potasse à 6 % (p/v) à une solution de 1,24 g (3 mmoles) de méthoxycarbonyl-5 diméthyl-2,2 phényl-4 (trichloro-2,2,2 éthoxycarbonyl)-3 oxazolidine-1,3 (4S,5R). Le mélange réactionnel est maintenu pendant 4 heures, sous agitation, à une température voisine de 20°C. Après addition de 5 cm3 d'eau et agitation pendant 30 minutes à une température voisine de 20°C, le mélange réactionnel est concentré à sec. Le résidu huileux est repris par 20 cm3 d'eau et extrait par 2 fois 20 cm3 d'éther diisopropylique. La phase aqueuse est acidifiée par addition d'une solution aqueuse d'acide chlorhydrique 1N jusqu'à pH = 2 puis est extraite par 20 cm3 de chlorure de méthylène. La phase organique est séchée puis concentrée sous pression réduite. On obtient ainsi, avec un rendement de 96 %, 1,15 g de carboxy-5 diméthyl-2,2 phényl-4 (trichloro-2,2,2 éthoxycarbonyl)-3 oxazolidine-1,3 (4S,5R).

Des résultats analogues sont obtenus en utilisant la méthoxycarbonyl-5 méthyl-2 isobutényl-2 phényl-4 (trichloro-2,2,2 éthoxycarbonyl)-3 oxazolidine-11,3 (4S,5R).

### EXEMPLE 3

A une solution agitée de 0,95 g de carboxy-5 diméthyl-2,2 phényl-4 (trichloro-2,2,2 éthoxycarbonyl)-3 oxazolidine-1,3 (4S,5R) et de 1,43 g d'acétoxy-4 benzoyloxy-2α époxy-5β,20 dihydroxy-1β,13α oxo-9 bis-(trichloro-2,2,2 éthoxycarbonyloxy)-7β,10β taxène-11 et de 0,039 g de diméthylamino-4 pyridine dans 20 cm3 de toluène anhydre, on ajoute à une température voisine de 20°C, 0,495 g de dicyclohexylcarbodiimide. Le mélange réactionnel est maintenu sous agitation pendant 2 heures. La dicyclohexylurée est séparée par filtration puis lavée par le toluène. Les phases toluéniques sont rassemblées, lavées successivement avec une solution aqueuse d'acide chlorhydrique 0,1N, une solution aqueuse saturée de bicarbonate de sodium, séchées sur sulfate de sodium, filtrées puis concentrées à sec sous pression réduite. On obtient ainsi, avec un rendement voisin de 78 %, 2,15 g de (trichloro-2,2,2 éthoxycarbonyl)-3 diméthyl-2,2 phényl-4 oxazolidinecarboxylate-5-(4S,5R) d'acétoxy-4 benzoyloxy-2α époxy-5β,20 hydroxy-1 oxo-9 bis-(trichloro-2,2,2 éthoxycarbonyloxy)-7β,10β taxène-11 yle-13α.

### EXEMPLE 4

A une solution agitée de 1,28 g du produit obtenu à l'exemple 3 dans 5 cm3 d'acétate d'éthyle, on ajoute 0,65 g de zinc en poudre puis, en 5 minutes, goutte à goutte, 1,8 cm3 d'acide acétique glacial. On note une légère exothermie et un dégagement gazeux. Le mélange réactionnel est alors maintenu à 45°C pendant 90 minutes puis refroidi à une température voisine de 20°C. Le zinc est séparé par filtration puis lavé à l'acétate d'éthyle. Les phases organiques réunies sont concentrées à sec sous pression réduite. Le résidu est repris par du toluène. La solution obtenue est à nouveau concentrée à sec sous pression réduite. Cette opération est répétée une première fois avec de l'heptane puis avec de l'acétate d'éthyle. Le résidu est repris à l'acétate d'éthyle. Cette solution est alors extraite par 10 cm3 d'une solution aqueuse d'acide chlorhydrique 0,1N. La phase aqueuse est neutralisée par addition d'une solution de soude 1N. On ajoute 10 cm3 d'acétate d'éthyle puis on ajuste le pH à 8 par addition d'une solution aqueuse saturée de bicarbonate de sodium. Après séparation, la phase aqueuse est extraite 2 fois par 25 cm3 d'acétate d'éthyle. Les phases organiques sont réunies, séchées sur sulfate de sodium, filtrées puis concentrées à sec sous pression réduite. On obtient ainsi, avec un rendement de 49 %, 0,355 g d'amino-3 hydroxy-2 phényl-3 propionate (2R,3S) d'acétoxy-4 benzoyloxy-2α époxy-5β,20 trihydroxy-1β,7α,10α oxo-9 taxène-11 yle-13α.

### EXEMPLE 5

A une solution de 0,3 g du produit obtenu à l'exemple 4 dans 5 cm3 de méthanol, on ajoute 0,108 g de dicarbonate de di-tert.butyle. Le mélange réactionnel est maintenu sous agitation pendant 15 heures à une température voisine de 20°C. Après addition de 20 cm3 d'eau, le mélange réactionnel est extrait par 3 fois 15 cm3 de chlorure de méthylène. Les phases aqueuses sont réunies, séchées sur sulfate de sodium puis concentrées à sec sous pression réduite. On obtient ainsi, avec un rendement de 70 %, 0,395 g de tert.butoxycarbonylamino-3 hydroxy-2 phényl-3 propionate (2R,3S) d'acétoxy-4 benzoyloxy-2α époxy-5β,20 trihydroxy-1,7β,10β oxo-9 taxène-11 yle-13α.

## Revendications

1. Procédé de préparation de dérivés du taxane de formule générale : 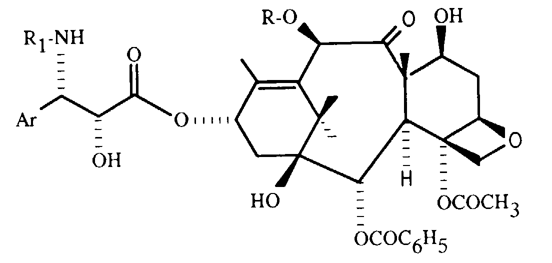 dans laquelle :
R représente un atome d'hydrogène ou un radical acétyle, R₁ représente un radical benzoyle ou un radical R₂-O-CO- dans lequel R₂ représente :
- un radical alcoyle droit ou ramifié contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone ou bicycloalcoyle contenant 7 à 10 atomes de carbone, ces radicaux étant éventuellement substitués par un ou plusieurs substituants choisis parmi les atomes d'halogène et les radicaux hydroxy, alcoyloxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle, cyano, carboxy ou alcoyloxycarbonyle dont la partie alcoyle contient 1 à 4 atomes de carbone,
- ou un radical phényle éventuellement substitué par un ou plusieurs atomes ou radicaux choisis parmi les radicaux alcoyles contenant 1 à 4 atomes de carbone ou alcoyloxy contenant 1 à 4 atomes de carbone,
- ou un radical hétérocyclique azoté saturé ou non saturé contenant 5 ou 6 chaînons éventuellement substitué par un ou plusieurs radicaux alcoyles contenant 1 à 4 atomes de carbone,
étant entendu que les radicaux cycloalcoyles, cycloalcényles ou bicycloalcoyles peuvent être éventuellement substitués par un ou plusieurs radicaux alcoyles contenant 1 à 4 atomes de carbone, et
Ar représente un radical phényle ou α- ou β-naphtyle éventuellement substitué par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alkylamino, dialkylamino, carboxy, alcoxycarbonyle, carbamoyle, dialcoylcarbamoyle, cyano et trifluorométhyle, étant entendu que les radicaux alcoyles et les portions alcoyles des autres radicaux contiennent 1 à 4 atomes de carbone, que les radicaux alcényles et alcynyles contiennent 3 à 8 atomes de carbone et les radicaux aryles sont les radicaux phényles ou α- ou β-naphtyles,
caractérisé en ce que :
1) on estérifie un dérivé de la baccatine III ou de la désacétyl-10 baccatine III protégée de formule générale : 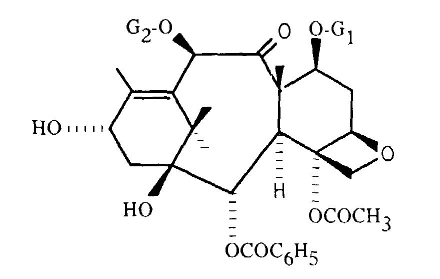 dans laquelle G₁ et éventuellement G₂ représentent un groupement protecteur de la fonction hydroxy, au moyen d'un acide de formule générale : 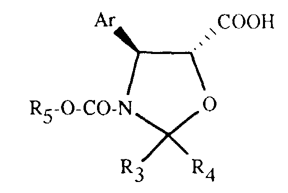 dans laquelle Ar est défini comme précédemment, R₃ et R₄, identiques ou différents, représentent un atome d'hydrogène ou un radical alcoyle contenant 1 à 4 atomes de carbone, ou un radical alcényle contenant 2 à 4 atomes de carbone, ou un radical aralcoyle dont la partie alcoyle contient 1 à 4 atomes de carbone et la partie aryle représente un radical phényle éventuellement substitué par un ou plusieurs radicaux alcoxy contenant 1 à 4 atomes de carbone, ou un radical aryle représentant un radical phényle éventuellement substitué par un ou plusieurs radicaux alcoxy contenant 1 à 4 atomes de carbone, ou bien R₃ et R₄ forment ensemble avec l'atome de carbone auquel ils sont liés un cycle ayant de 4 à 7 chaînons, étant entendu que R₃ et R₄ ne représentent pas simultanément un atome d'hydrogène et que lorque l'un des symboles R₃ et R₄ représente un atome d'hydrogène, l'autre ne peut pas représenter un radical phényle substitué par un ou plusieurs radicaux alcoxy contenant 1 à 4 atomes de carbone ou par un radical trihalométhyle, et R₅ représente un radical alcoyle contenant 1 à 4 atomes de carbone substitué par un ou plusieurs atomes de chlore, ou un dérivé activé de cet acide, pour obtenir un produit de formule générale : 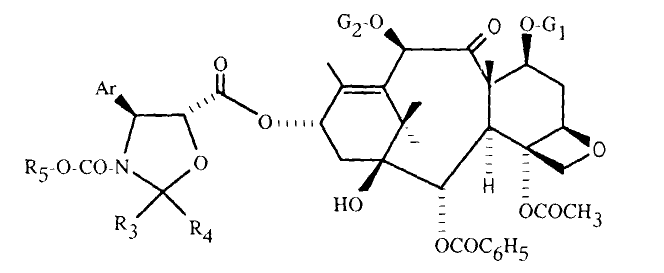 dans laquelle Ar, R₃, R₄, R₅, G₁ et G₂ sont définis comme précédemment,
b) on remplace les groupements protecteurs des fonctions hydroxy et amino du produit obtenu par des atomes d'hydrogène, pour obtenir un produit de formule générale : 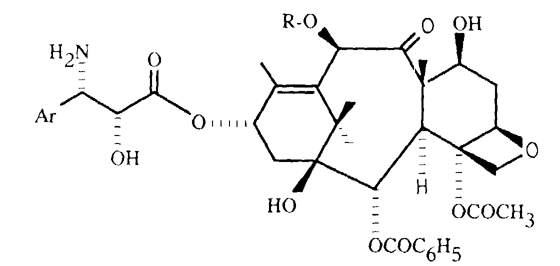 dans laquelle Ar et R sont définis comme précédemment puis
c) traite le produit ainsi obtenu par un réactif permettant d'introduire un substituant R₁ sur la fonction amino, et
d) isole le produit obtenu.

2. Procédé selon la revendication 1 caractérisé en ce que l'estérification est effectuée au moyen d'un acide de formule générale : 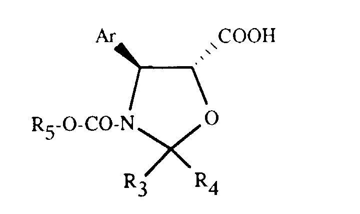 dans laquelle Ar, R₃, R₄ et R₅ sont définis comme dans la revendication 1 en opérant en présence d'un agent de condensation et d'un agent d'activation dans un solvant organique à une température comprise entre -10 et 90°C.

3. Procédé selon la revendication 2 caractérisé en ce que l'agent de condensation est choisi parmi les imides et les carbonates réactifs et l'agent d'activation est choisi parmi les aminopyridines.

4. Procédé selon la revendication 3 caractérisé en ce que l'agent de condensation est choisi parmi le dicyclohexylcarbodiimide et le dipyridyl-2 carbonate et l'agent d'activation est choisi parmi la diméthylamino-4 pyridine ou la pyrrolidino-4 pyridine.

5. Procédé selon la revendication 2 caractérisé en ce que le solvant est choisi parmi les éthers, les cétones, les esters, les nitriles, les hydrocarbures aliphatiques, les hydrocarbures aliphatiques halogénés et les hydrocarbures aromatiques.

6. Procédé selon la revendication 5 caractérisé en ce que le solvant est choisi parmi les hydrocarbures aromatiques.

7. Procédé selon la revendication 1 caractérisé en ce que l'estérification est effectuée au moyen d'un anhydride de formule générale : 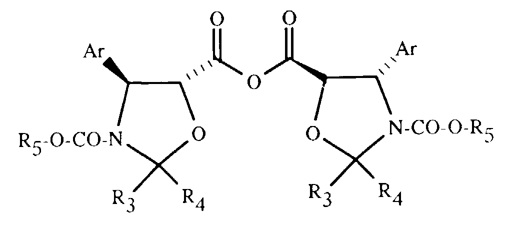 dans laquelle Ar, R₃, R₄ et R₅ sont définis comme dans la revendication 1 en opérant en présence d'un agent d'activation dans un solvant organique à une température comprise entre 0 et 90°C.

8. Procédé selon la revendication 7 caractérisé en ce que l'agent d'activation est choisi parmi les aminopyridines.

9. Procédé selon la revendication 8 caractérisé en ce que l'agent d'activation est choisi parmi la diméthylamino-4 pyridine ou la pyrrolidino-4 pyridine.

10. Procédé selon la revendication 7 caractérisé en ce que le solvant est choisi parmi les éthers, les cétones, les esters, les nitriles, les hydrocarbures aliphatiques, les hydrocarbures aliphatiques halogénés et les hydrocarbures aromatiques.

11. Procédé selon la revendication 1 caractérisé en ce que l'estérification est effectuée au moyen d'un acide activé de formule générale : 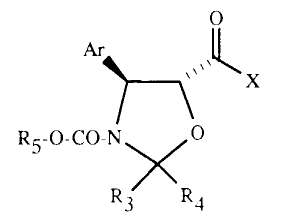 dans laquelle Ar, R₃, R₄ et R₅ sont définis comme précédemment et X représente un atome d'halogène ou un radical acyloxy ou aroyloxy, éventuellement préparé in situ, en présence d'une base en opérant dans un solvant organique à une température comprise entre 10 et 80°C.

12. Procédé selon la revendication 11 caractérisé en ce que la base est choisie parmi les bases organiques azotées.

13. Procédé selon la revendication 12 caractérisé en ce que la base organique azotée est choisie parmi les amines tertiaires aliphatiques, la pyridine et les aminopyridines.

14. Procédé selon la revendication 11 caractérisé en ce que le solvant organique est choisi parmi les éthers, les cétones, les esters, les nitriles, les hydrocarbures aliphatiques, les hydrocarbures aliphatiques halogénés et les hydrocarbures aromatiques.

15. Procédé selon la revendication 14 caractérisé en ce que le solvant est choisi parmi les hydrocarbures aromatiques.

16. Procédé selon la revendication 1 caractérisé en ce que le remplacement par des atomes d'hydrogène des groupements protecteurs des fonctions hydroxy et amino est effectué par traitement par le zinc, éventuellement associé à du cuivre, en présence d'acide acétique à une température comprise entre 30 et 60°C.

17. Procédé selon la revendication 1 caractérisé en ce que le remplacement par des atomes d'hydrogène des groupements protecteurs de la fonction hydroxy et amino est effectué au moyen d'un acide minéral ou organique en solution dans un alcool aliphatique contenant 1 à 3 atomes de carbone ou dans un ester aliphatique en présence de zinc éventuellement associé à du cuivre.

18. Procédé selon l'une des revendications 16 ou 17 dans lequel R₅ représente un radical trichloro-2,2,2 éthyle ou (trichlorométhyl-2 propyle)-2 et G₁ et éventuellement G₂ représentent un radical trichloro-2,2,2 éthoxycarbonyle ou (trichlorométhyl-2 propoxy)-2 carbonyle.

19. Procédé selon la revendication 1 caractérisé en ce que l'introduction d'un substituant R₁ sur la fonction amino est effectuée par action du chlorure de benzoyle ou d'un dérivé réactif de formule générale :
R₂-O-CO-Y
dans laquelle Y représente un atome d'halogène et R₂ est défini comme dans la revendication 1 en opérant dans un solvant organique en présence d'une base minérale ou organique à une température comprise entre 0 et 50°C.

20. Procédé selon la revendication 19 caractérisé en ce que le solvant est choisi parmi les alcools, les esters aliphatiques et les hydrocarbures aliphatiques halogénés.

21. Procédé selon la revendication 19 caractérisé en ce que la base est le bicarbonate de sodium.

22. Les acides de formule générale : 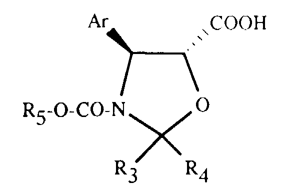 dans laquelle Ar, R₃, R₄ et R₅ sont définis comme dans la revendication 1, éventuellement sous forme de sel, d'ester, d'anhydride, d'anhydride mixte ou d'halogénure.

23. Un produit de formule générale : 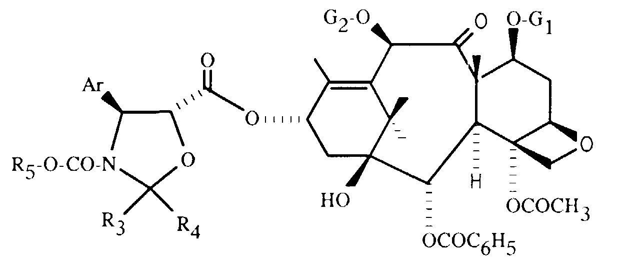 dans laquelle Ar, R₃, R₄, R₅, G₁ et G₂ sont définis comme dans la revendication 1.

24. Un produit selon la revendication 23 dans lequel Ar, R₃ et R₄ étant définis comme dans la revendication 1, R₅ représente un radical trichloro-2,2,2 éthyle ou trichlorométhyl-2 isopropyle et G₁ représente un radical trichloro-2,2,2 éthoxycarbonyle ou (trichlorométhyl-2 propoxy)-2 carbonyle et G₂ représente un radical acétyle ou un radical trichloro-2,2,2 éthoxycarbonyle ou (trichlorométhyl-2 propoxy)-2 carbonyle.

## Patentansprüche

1. Verfahren zur Herstellung von Taxan-Derivaten der allgemeinen Formel 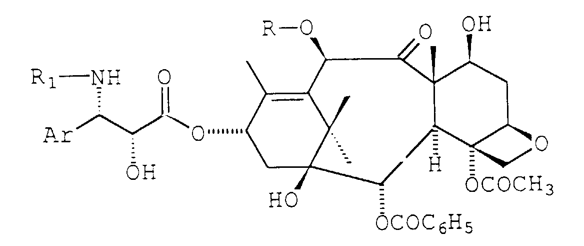 in der
R ein Wasserstoffatom oder einen Acetylrest darstellt, R₁ einen Benzoylrest oder einen Rest R₂-O-CO- bedeutet, worin R₂ ist:
- ein gerader oder verzweigter Rest Alkyl mit 1 bis 8 Kohlenstoffatomen, Alkenyl mit 2 bis 8 Kohlenstoffatomen, Alkinyl mit 3 bis 8 Kohlenstoffatomen, Cycloalkyl mit 3 bis 6 Kohlenstoffatomen, Cycloalkenyl mit 4 bis 6 Kohlenstoffatomen oder Bicycloalkyl mit 7 bis 10 Kohlenstoffatomen, wobei diese Reste gegebenenfalls substituiert sind durch einen oder mehrere Substituenten, ausgewählt unter den Halogenatomen und den Resten Hydroxy, Alkyloxy mit 1 bis 4 Kohlenstoffatomen, Dialkylamino, dessen Alkylteil 1 bis 4 Kohlenstoffatome enthält, Piperidino, Morpholino, 1-Piperazinyl (gegebenenfalls substituiert in 4 durch einen Alkylrest mit 1 bis 4 Kohlenstoffatomen oder durch einen Phenylalkylrest, dessen Alkylteil 1 bis 4 Kohlenstoffatome enthält), Cycloalkyl mit 3 bis 6 Kohlenstoffatomen, Cycloalkenyl mit 4 bis 6 Kohlenstoffatomen, Phenyl, Cyano, Carboxy oder Alkyloxycarbonyl, dessen Alkylteil 1 bis 4 Kohlenstoffatome enthält,
- oder ein Rest Phenyl, gegebenenfalls substituiert durch ein oder mehrere Atome oder Reste, ausgewählt unter den Alkylresten mit 1 bis 4 Kohlenstoffatomen oder den Alkoxyresten mit 1 bis 4 Kohlenstoffatomen,
- oder ein Rest eines gesättigten oder ungesättigten Stickstoff-Heterocyclus mit 5 oder 6 Ringgliedern, gegebenenfalls substituiert durch einen oder mehrere Alkylreste mit 1 bis 4 Kohlenstoffatomen,
mit der Maßgabe, daß die Reste Cycloalkyl, Cycloalkenyl oder Bicycloalkyl gegebenenfalls durch einen oder mehrere Alkylreste mit 1 bis 4 Kohlenstoffatomen substituiert sein können, und Ar einen Rest Phenyl oder α- oder ß-Naphthyl darstellt, gegebenenfalls substituiert durch ein oder mehrere Atome oder Reste, ausgewählt unter den Halogenatomen (Fluor, Chlor, Brom, Iod) und den Resten Alkyl, Alkenyl, Alkinyl, Aryl, Arylalkyl, Alkoxy, Alkylthio, Aryloxy, Arylthio, Hydroxy, Hydroxyalkyl, Mercapto, Formyl, Acyl, Acylamino, Arylamino, Alkoxycarbonylamino, Amino, Alkylamino, Dialkylamino, Carboxy, Alkoxycarbonyl, Carbamoyl, Dialkylcarbamoyl, Cyano und Trifluormethyl, mit der Maßgabe, daß die Alkylreste und Alkylteile der anderen Reste 1 bis 4 Kohlenstoffatome, die Reste Alkenyl und Alkinyl 3 bis 8 Kohlenstoffatome enthalten und die Arylreste Phenylreste oder α- oder β-Naphthylreste sind,
dadurch gekennzeichnet, daß man
a) ein geschütztes Derivat von Baccatin III oder von 10-Desacetyl-Baccatin III der allgemeinen Formel 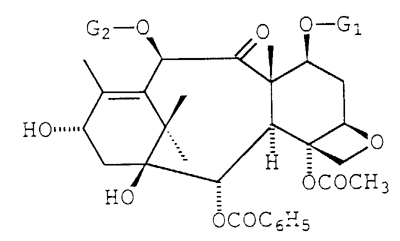 in der G₁ und gegebenenfalls G₂ eine Schutzgruppe für die Hydroxyfunktion darstellt, mit Hilfe einer Säure der allgemeinen Formel 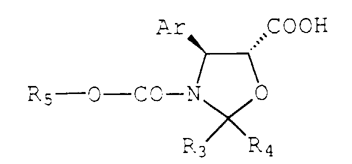 oder einem aktivierten Derivat dieser Säure verestert, worin Ar wie oben definiert ist, R₃ und R₄, gleich oder verschieden, darstellen: ein Wasserstoffatom oder einen Alkylrest mit 1 bis 4 Kohlenstoffatomen, oder einen Alkenylrest mit 2 bis 4 Kohlenstoffatomen, oder einen Aralkylrest, dessen Alkylteil 1 bis 4 Kohlenstoffatome enthält und der Arylteil einen Phenylrest, gegebenenfalls substituiert durch einen oder mehrere Alkylreste mit 1 bis 4 Kohlenstoffatomen, oder einen Arylrest bedeutet, der ein Phenylrest ist, gegebenenfalls substituiert durch einen oder mehrere Alkylreste mit 1 bis 4 Kohlenstoffatomen,
oder auch R₃ und R₄ zusammen mit dem Kohlenstoffatom, an das sie gebunden sind, einen Ring mit 4 bis 7 Ringgliedern bilden, mit der Maßgabe, daß R₃ und R₄ nicht gleichzeitig ein Wasserstoffatom darstellen und daß, wenn eines der Symbole R₃ und R₄ ein Wasserstoffatom ist, das andere keinen Phenylrest bedeuten kann,
substituiert durch einen oder mehrere Alkoxyreste mit 1 bis 4 Kohlenstoffatomen oder durch einen Rest Trihalomethyl, und R₅ einen Alkylrest mit 1 bis 4 Kohlenstoffatomen darstellt, substituiert durch ein oder mehrere Chloratome, um ein Produkt der allgemeinen Formel 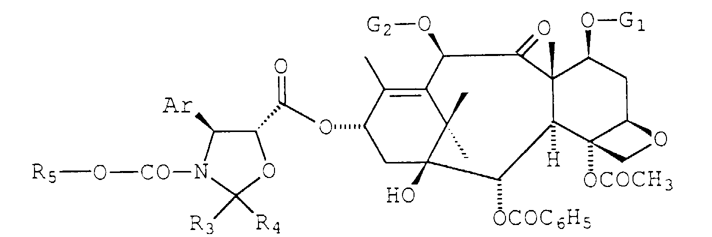 zu erhalten, worin Ar, R₃, R₄, R₅, G₁ und G₂ wie oben definiert sind,
b) die Schutzgruppen für die Funktionen Hydroxy und Amino des erhaltenen Produktes durch Wasserstoffatome ersetzt, um ein Produkt der allgemeinen Formel 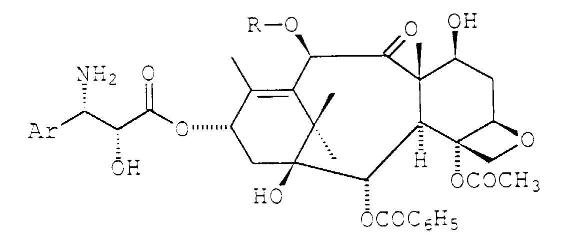 zu erhalten, worin Ar und R wie oben definiert sind, und
c) anschließend das auf diese Weise erhaltene Produkt mit einem Reaktanden behandelt, der ermöglicht, einen Substituenten R₁ bei der Aminfunktion einzuführen, und
d) das erhaltene Produkt isoliert.

2. Verfahren nach Anspruch 1, dadurch gekennzeichnet, daß die Veresterung mit Hilfe einer Säure der allgemeinen Formel 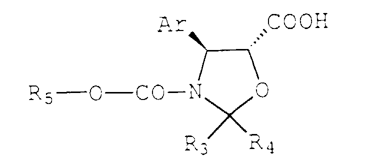 durchgeführt wird, in der Ar, R₃, R₄ und R₅ wie in Anspruch 1 definiert sind, wobei man in Anwesenheit eines Kondensationsmittels und eines Aktivierungsmittels in einem organischen Lösungsmittel bei einer Temperatur zwischen -10 °C und 90 °C arbeitet.

3. Verfahren nach Anspruch 2, dadurch gekennzeichnet, daß das Kondensationsmittel unter den reaktiven Imiden und den Carbonaten und das Aktivierungsmittel unter den Aminopyridinen ausgewählt werden.

4. Verfahren nach Anspruch 3, dadurch gekennzeichnet, daß das Kondensationsmittel unter Dicyclohexylcarbodiimid und 2-Dipyridylcarbonat und das Aktivierungsmittel unter 4-Dimethylaminopyridin oder 4-Pyrrolidinopyridin ausgewählt werden.

5. Verfahren nach Anspruch 2, dadurch gekennzeichnet, daß das Lösungsmittel unter den Ethern, den Ketonen, den Estern, den Nitrilen, den aliphatischen Kohlenwasserstoffen, den halogenierten aliphatischen Kohlenwasserstoffen und den aromatischen Kohlenwasserstoffen ausgewählt wird.

6. Verfahren nach Anspruch 5, dadurch gekennzeichnet, daß das Lösungsmittel unter den aromatischen Kohlenwasserstoffen ausgewählt wird.

7. Verfahren nach Anspruch 1, dadurch gekennzeichnet, daß die Veresterung mit Hilfe eines Anhydrides der allgemeinen Formel 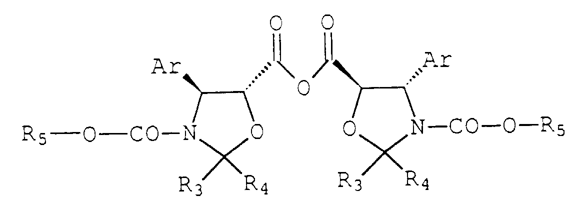 durchgeführt wird, in der Ar, R₃, R₄ und R₅ wie in Anspruch 1 definiert sind, wobei man in Anwesenheit eines Aktivierungsmittels in einem organischen Lösungsmittel bei einer Temperatur zwischen 0 °C und 90 °C arbeitet.

8. Verfahren nach Anspruch 7, dadurch gekennzeichnet, daß das Aktivierungsmittel unter den Aminopyridinen ausgewählt wird.

9. Verfahren nach Anspruch 8, dadurch gekennzeichnet, daß das Aktivierungsmittel unter 4-Dimethylaminopyridin oder 4-Pyrrolidinopyridin ausgewählt wird.

10. Verfahren nach Anspruch 7, dadurch gekennzeichnet, daß das Lösungsmittel unter den Ethern, den Ketonen, den Estern, den Nitrilen, den aliphatischen Kohlenwasserstoffen, den halogenierten aliphatischen Kohlenwasserstoffen und den aromatischen Kohlenwasserstoffen ausgewählt wird.

11. Verfahren nach Anspruch 1, dadurch gekennzeichnet, daß die Veresterung mit Hilfe einer aktivierten Säure der allgemeinen Formel 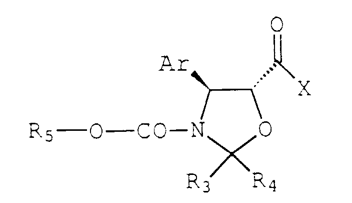 gegebenenfalls hergestellt in situ, durchgeführt wird, in der Ar, R₃, R₄ und R₅ wie oben definiert sind und X ein Halogenatom oder einen Rest Acyloxy oder Aryloxy darstellt, wobei man in Anwesenheit einer Base in einem organischen Lösungsmittel und bei einer Temperatur zwischen 10 °C und 80 °C arbeitet.

12. Verfahren nach Anspruch 11, dadurch gekennzeichnet, daß die Base unter den organischen Stickstoffbasen ausgewählt wird.

13. Verfahren nach Anspruch 12, dadurch gekennzeichnet, daß die organische Stickstoffbase unter den tertiären aliphatischen Aminen, Pyridin und den Aminopyridinen ausgewählt wird.

14. Verfahren nach Anspruch 11, dadurch gekennzeichnet, daß das organische Lösungsmittel unter den Ethern, den Ketonen, den Estern, den Nitrilen, den aliphatischen Kohlenwasserstoffen, den halogenierten aliphatischen Kohlenwasserstoffen und den aromatischen Kohlenwasserstoffen ausgewählt wird.

15. Verfahren nach Anspruch 14, dadurch gekennzeichnet, daß das Lösungsmittel unter den aromatischen Kohlenwasserstoffen ausgewählt wird.

16. Verfahren nach Anspruch 1, dadurch gekennzeichnet, daß der Austausch der Schutzgruppen für die Hydroxyfunktion und die Aminfunktion durch Wasserstoffatome mit Hilfe einer Behandlung durch Zink, gegebenenfalls assoziiert mit Kupfer, in Anwesenheit von Essigsäure bei einer Temperatur zwischen 30 °C und 60 °C durchgeführt wird.

17. Verfahren nach Anspruch 1, dadurch gekennzeichnet, daß der Austausch der Schutzgruppen für die Hydroxyfunktion und die Aminfunktion durch Wasserstoffatome mit Hilfe einer Mineralsäure oder organischen Säure in Lösung eines aliphatischen Alkohols mit 1 bis 3 Kohlenstoffatomen oder in einem aliphatischen Ester in Anwesenheit von Zink, gegebenenfalls assoziiert mit Kupfer, durchgeführt wird.

18. Verfahren nach einem der Ansprüche 16 oder 17, worin R₅ einen Rest 2,2,2-Trichlorethyl oder 2-(2-Trichlormethyl-propyl) darstellt und G₁ und gegebenenfalls G₂ einen Rest 2,2,2-Trichlorethoxycarbonyl oder 2-(2-Trichlormethyl-propoxy)-carbonyl bedeuten.

19. Verfahren nach Anspruch 1, dadurch gekennzeichnet, daß die Einführung eines Substituenten R₁ bei der Aminfunktion durch Umsetzung mit Benzoylchlorid oder einem reaktiven Derivat der allgemeinen Formel
R₂-O-CO-Y
durchgeführt wird, worin Y ein Halogenatom darstellt und R₂ wie in Anspruch 1 definiert ist, wobei man in einem organischen Lösungsmittel in Anwesenheit einer Mineralbase oder organischen Base und bei einer Temperatur zwischen 0 °C und 50 °C arbeitet.

20. Verfahren nach Anspruch 19, dadurch gekennzeichnet, daß das organische Lösungsmittel unter den Alkoholen, den aliphatischen Estern und den halogenierten aliphatischen Kohlenwasserstoffen ausgewählt wird.

21. Verfahren nach Anspruch 19, dadurch gekennzeichnet, daß die Base Natriumbicarbonat ist.

22. Die Säuren der allgemeinen Formel 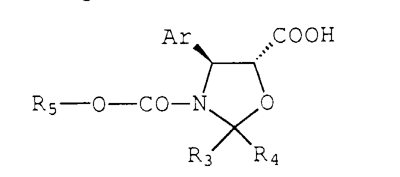 worin Ar, R₃, R₄ und R₅ wie in Anspruch 1 definiert sind, gegebenenfalls in Form von Salz, Ester, Anhydrid, gemischtes Anhydrid oder Halogenid.

23. Ein Produkt der allgemeinen Formel 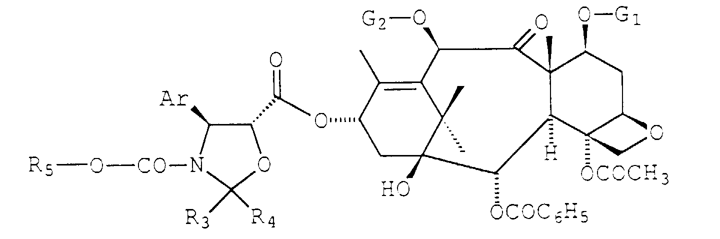 worin Ar, R₃, R₄, R₅, G₁ und G₂ wie in Anspruch 1 definiert sind.

24. Ein Produkt nach Anspruch 23, worin Ar, R₃ und R₄ wie in Anspruch 1 definiert sind, R₅ einen Rest 2,2,2-Trichlorethyl oder 2Trichlormethyl-isopropyl darstellt, G₁ einen Rest 2,2,2-Trichlorethoxycarbonyl oder 2-(2-Trichlormethyl-propoxy)-carbonyl bedeutet und G₂ ein Acetylrest oder ein Rest 2,2,2-Trichlor-ethoxycarbonyl oder 2-(2-Trichlormethyl-propoxy)-carbonyl ist.

## Claims

1. Process for preparing taxane derivatives of general formula: 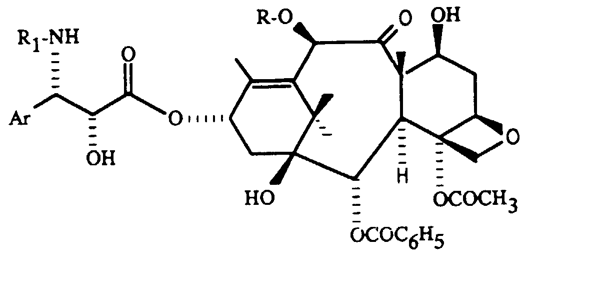 in which:
R represents a hydrogen atom or an acetyl radical, and R₁ represents a benzoyl radical or a radical R₂-O-CO- in which R₂ represents:
- an unbranched or branched alkyl radical containing 1 to 8 carbon atoms, an alkenyl radical containing 2 to 8 carbon atoms, an alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals being optionally substituted with one or more substituents chosen from halogen atoms and hydroxyl radicals, alkyloxy radicals containing 1 to 4 carbon atoms, dialkylamino radicals in which each alkyl portion contains 1 to 4 carbon atoms, piperidino radicals, morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl, cyano or carboxyl radicals or alkyloxycarbonyl radicals in which the alkyl portion contains 1 to 4 carbon atoms,
- or a phenyl radical optionally substituted with one or more atoms or radicals chosen from alkyl radicals containing 1 to 4 carbon atoms or alkyloxy radicals containing 1 to 4 carbon atoms,
- or a saturated or unsaturated 5- or 6-membered nitrogenous heterocyclic radical optionally substituted with one or more alkyl radicals containing 1 to 4 carbon atoms,
on the understanding that the cycloalkyl, cycloalkenyl or bicycloalkyl radicals can be optionally substituted with one or more alkyl radicals containing 1 to 4 carbon atoms, and
Ar represents a phenyl or α- or β-naphthyl radical optionally substituted with one or more atoms or radicals chosen from halogen (fluorine, chlorine, bromine, iodine) atoms and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, dialkylcarbamoyl, cyano and trifluoromethyl radicals, on the understanding that the alkyl radicals and alkyl portions of the other radicals contain 1 to 4 carbon atoms, and that the alkenyl and alkynyl radicals contain 3 to 8 carbon atoms and the aryl radicals are phenyl or α- or β-naphthyl radicals,
characterized in that:
a) a protected baccatin III or 10-deacetylbaccatin III derivative of general formula: 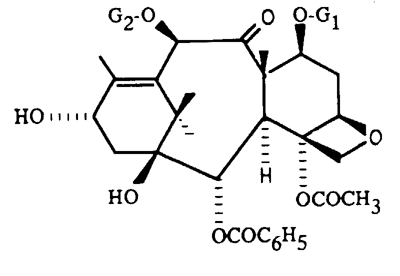 in which G₁ and, where appropriate, G₂ represent a group protecting the hydroxyl function, is esterified by means of an acid of general formula: 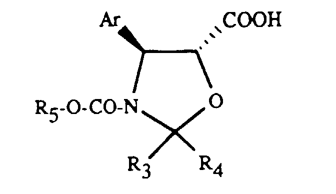 in which Ar is defined as above, R₃ and R₄, which may be identical or different, represent a hydrogen atom or an alkyl radical containing 1 to 4 carbon atoms or an alkenyl radical containing 2 to 4 carbon atoms, or an aralkyl radical in which the alkyl portion contains 1 to 4 carbon atoms and the aryl portion represents a phenyl radical optionally substituted with one or more alkoxy radicals containing 1 to 4 carbon atoms, or an aryl radical representing a phenyl radical optionally substituted with one or more alkoxy radicals containing 1 to 4 carbon atoms, or alternatively R₃ and R₄, together with the carbon atom to which they are linked, form a 4- to 7-membered ring, on the understanding that R₃ and R₄ do not simultaneously represent a hydrogen atom and that, when one of the symbols R₃ and R₄ represents a hydrogen atom, the other cannot represent a phenyl radical substituted with one or more alkoxy radicals containing 1 to 4 carbon atoms or with a trihalomethyl radical, and R₅ represents an alkyl radical containing 1 to 4 carbon atoms substituted with one or more chlorine atoms, or an activated derivative of this acid, to obtain a product of general formula: 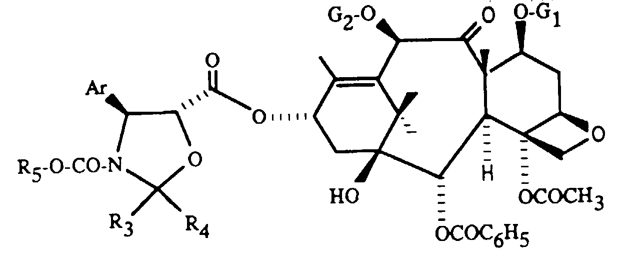 in which Ar, R₃, R₄, R₅, G₁ and G₂ are defined as above,
b) the groups protecting the hydroxyl and amino functions of the product obtained are replaced by hydrogen atoms to obtain a product of general formula: 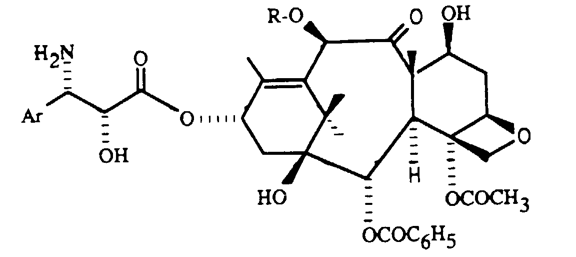 in which Ar and R are defined as above, then
c) the product thereby obtained is treated with a reagent which enables a substituent R₁ to be introduced on the amino function, and
d) the product obtained is isolated.

2. Process according to claim 1, characterized in that the esterification is performed by means of an acid of general formula: 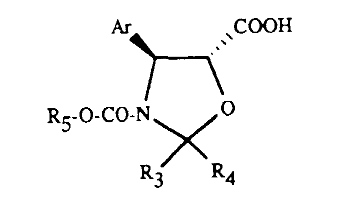 in which Ar, R₃, R₄ and R₅ are defined as in claim 1, working in the presence of a condensing agent and an activating agent in an organic solvent at a temperature of between -10 and 90°C.

3. Process according to claim 2, characterized in that the condensing agent is chosen from imides and reactive carbonates and the activating agent is chosen from aminopyridines.

4. Process according to claim 3, characterized in that the condensing agent is chosen from dicyclohexylcarbodiimide and di-2-pyridyl carbonate and the activating agent is chosen from 4-(dimethylamino)pyridine and 4-pyrrolidinopyridine.

5. Process according claim 2, characterized in that the solvent is chosen from ethers, ketones, esters, nitriles, aliphatic hydrocarbons, halogenated aliphatic hydrocarbons and aromatic hydrocarbons.

6. Process according to claim 5, characterized in that the solvent is chosen from aromatic hydrocarbons.

7. Process according to claim 1, characterized in that the esterification is performed by means of an anhydride of general formula: 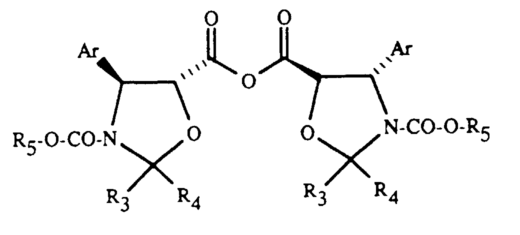 in which Ar, R₃, R₄ and R₅ are defined as in claim 1, working in the presence of an activating agent in an organic solvent at a temperature of between 0 and 90°C.

8. Process according to claim 7, characterized in that the activating agent is chosen from aminopyridines.

9. Process according to claim 8, characterized in that the activating agent is chosen from 4-(dimethylamino)pyridine and 4-pyrrolidinopyridine.

10. Process according to claim 7, characterized in that the solvent is chosen from ethers, ketones, esters, nitriles, aliphatic hydrocarbons, halogenated aliphatic hydrocarbons and aromatic hydrocarbons.

11. Process according to claim 1, characterized in that the esterification is performed by means of an activated acid of general formula: 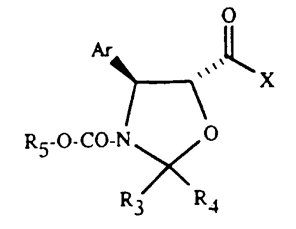 in which Ar, R₃, R₄ and R₅ are defined as above and X represents a halogen atom or an acyloxy or aroyloxy radical, optionally prepared in situ, in the presence of a base, working in an organic solvent at a temperature of between 10 and 80°C.

12. Process according to claim 11, characterized in that the base is chosen from nitrogenous organic bases.

13. Process according to claim 12, characterized in that the nitrogenous organic base is chosen from aliphatic tertiary amines, pyridine and aminopyridines.

14. Process according to claim 11, characterized in that the organic solvent is chosen from ethers, ketones, esters, nitriles, aliphatic hydrocarbons, halogenated aliphatic hydrocarbons and aromatic hydrocarbons.

15. Process according to claim 14, characterized in that the solvent is chosen from aromatic hydrocarbons.

16. Process according to claim 1, characterized in that the replacement by hydrogen atoms of the groups protecting the hydroxyl and amino functions is performed by treatment with zinc, optionally in combination with copper, in the presence of acetic acid at a temperature of between 30 and 60°C.

17. Process according to claim 1, characterized in that the replacement by hydrogen atoms of the groups protecting the hydroxyl and amino functions is performed by means of an inorganic or organic acid dissolved in an aliphatic alcohol containing 1 to 3 carbon atoms or in an aliphatic ester, in the presence of zinc optionally in combination with copper.

18. Process according to one of claims 16 and 17, in which R₅ represents a 2,2,2-trichloroethyl or 2-trichoromethyl-2-propyl radical and G₁ and, where appropriate, G₂ represent a 2,2,2-trichoroethoxycarbonyl or (2-trichloromethyl-2-propoxy)carbonyl radical.

19. Process according claim 1, characterized in that the introduction of a substituent R₁ on the amino function is performed by the action of benzoyl chloride or a reactive derivative of general formula:
R₂-O-CO-Y
in which Y represents a halogen atom and R₂ is defined as in claim 1, working in an organic solvent in the presence of an inorganic or organic base at a temperature of between 0 and 50°C.

20. Process according to claim 19, characterized in that the solvent is chosen from alcohols, aliphatic esters and halogenated aliphatic hydrocarbons.

21. Process according to claim 19, characterized in that the base is sodium bicarbonate.

22. The acids of general formula: 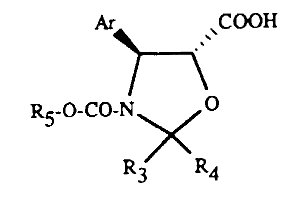 in which Ar, R₃, R₄ and R₅ are defined as in claim 1, optionally in the form of a salt, ester, anhydride, mixed anhydride or halide.

23. A product of general formula: 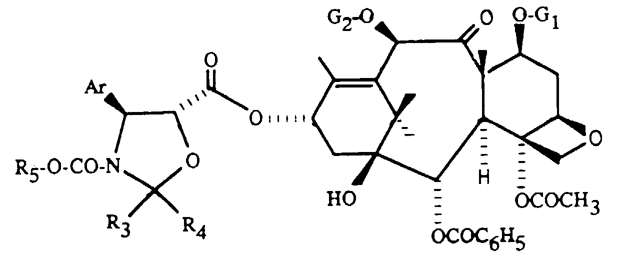 in which Ar, R₃, R₄, R₅, G₁ and G₂ are defined as in claim 1.

24. A product according to claim 23 in which, Ar, R₃ and R₄ being defined as in claim 1, R₅ represents a 2,2,2-trichloroethyl or 2-(trichloromethyl)isopropyl radical and G₁ represents a 2,2,2-trichloroethoxycarbonyl or (2-trichloromethyl-2-propoxy) carbonyl radical and G₂ represents an acetyl radical or a 2,2,2-trichloroethoxycarbonyl or (2-trichloromethyl-2propoxy)carbonyl radical.
